# FASCICULE DE BREVET EUROPEEN

(11) **EP 3 713 924 B1**
(45) Date de publication et mention de la délivrance du brevet: **04.01.2023**
(21) Numéro de dépôt: 18827180.3
(22) Date de dépôt: 20.11.2018
(51) Int. Cl.: C07D 303/38, C08C 19/06, C08F 236/08, C08F 236/10

(54) **COMPOSÉ 1,3-DIPOLAIRE COMPORTANT UN GROUPE ÉPOXYDE**
1,3-DIPOLARE VERBINDUNG MIT EINER EPOXIDGRUPPE
1,3-DIPOLAR COMPOUND COMPRISING AN EPOXIDE GROUP

(30) Priorité: 21.11.2017 FR 1760957
(43) Date de publication de la demande: 30.09.2020
(73) Titulaire: Compagnie Générale des Etablissements Michelin, 63000 Clermont-Ferrand (FR)
(72) Inventeur: SALIT, Anne-Frédérique, 63040 Clermont-Ferrand Cedex 9 (FR); SCHNELL, Benoît, 63040 Clermont-Ferrand Cedex 9 (FR); GANDER, Sophie, 63040 Clermont-Ferrand Cedex 9 (FR); IVANOV, Sergey, 63040 Clermont-Ferrand Cedex 9 (FR); FLEURY, Etienne, 63040 Clermont-Ferrand Cedex 9 (FR)
(74) Mandataire: Gandon-Pain, Sylvie
(86) Numéro de dépôt international: PCT/FR2018/052913
(87) Numéro de publication internationale: WO 2019/102128

(56) Documents cités:
- US-A- 3 941 751
- US-A1- 2012 046 418
- JACQUES M. LEMMENS ET AL: "Synthesis of .alpha.,.beta.-epoxyacyl azides and their rearrangement to epoxy isocyanates and 3- and 4-oxazolin-2-ones", JOURNAL OF ORGANIC CHEMISTRY, vol. 49, no. 12, 1 juin 1984 (1984-06-01), pages 2231-2235, XP055486649, ISSN: 0022-3263, DOI: 10.1021/jo00186a030
- H.-p Stupp ET AL: "ChemInform Abstract: Synthesis of Aryl Azide Analogues of Insect Juvenile Hormones as Reagents for the Photoaffinity Labeling of Juvenile Hormone Binding Proteins", ChemInform, 4 août 1987 (1987-08-04), pages no-no, XP055486651, Weinheim DOI: 10.1002/chin.198731329 Extrait de l'Internet: URL:https://onlinelibrary.wiley.com/doi/pd f/10.1002/jlac.198719870329
- STUART LANG ET AL: "Amination of Arenes through Electron-Deficient Reaction Cascades of Aryl Epoxyazides", ORGANIC LETTERS , 14(23), 6012-6015 CODEN: ORLEF7; ISSN: 1523-7052, vol. 5, no. 20, 1 octobre 2003 (2003-10-01), pages 3655-3658, XP055486652, ISSN: 1523-7060, DOI: 10.1021/ol035319d

## Description

Le domaine de la présente invention est celui des agents de modification de polymères diéniques, plus particulièrement celui des agents de modification pour greffer des fonctions époxyde sur un polymère diénique.

Il est connu de la demande de brevet US 2012/0046418 A1 de modifier un élastomère diénique par réaction d'un composé 1,3-dipolaire portant un groupe glycidyle sur les unités diéniques de l'élastomère. La modification conduit à l'obtention d'un élastomère diénique portant des groupes pendants glycidyle. Ces élastomères ainsi modifiés peuvent être utilisés réticulés dans une composition de caoutchouc, la présence des groupes pendants glycidyle permettant de réticuler l'élastomère diénique en présence d'un agent de réticulation autre que le soufre. Il s'avère que les élastomères ainsi modifiés confèrent à la composition de caoutchouc qui les contient des propriétés à la rupture dégradées.

Or une composition de caoutchouc réticulée doit présenter de bonnes propriétés à la rupture pour pouvoir être utilisée dans un article semi-fini pour pneumatique. En effet, le pneumatique au cours du roulage est soumis à de fortes contraintes et à de grandes déformations, sachant qu'il doit aussi présenter une résistance au roulement la plus faible possible.

La Demanderesse a découvert, de façon surprenante, que l'utilisation de composés 1,3-dipolaires monooxydes de nitrile aromatiques portant un groupe époxyde substitué particulier dans la modification d'élastomères diéniques permet d'améliorer les propriétés à la rupture d'une composition de caoutchouc comprenant de tels élastomères modifiés sans être au détriment de ses propriétés d'hystérèse tout en améliorant sa mise en œuvre.

Un premier objet de l'invention est un composé 1,3-dipolaire comportant un groupe époxyde, le groupe époxyde étant un cycle éther à 3 membres dans lequel un premier membre est un atome de carbone présentant un rattachement au dipôle du composé 1,3-dipolaire et un deuxième membre est un carbone tertiaire ou quaternaire, lequel composé étant un monooxyde de nitrile aromatique.

Un autre objet de l'invention est un procédé pour un polymère diénique, notamment un élastomère diénique, par une réaction de greffage qui comprend la réaction d'un polymère diénique de départ et d'un composé 1,3-dipolaire conforme à l'invention.

L'invention concerne aussi un polymère, notamment un élastomère diénique, susceptible d'être obtenu par le procédé conforme à l'invention.

L'invention concerne également une composition de caoutchouc qui comprend une charge renforçante, un système de réticulation et un élastomère diénique conforme à l'invention.

Un autre objet de l'invention est un pneumatique qui comprend une composition de caoutchouc conforme à l'invention.

### I. DESCRIPTION DETAILLEE DE L'INVENTION

Dans la présente description, sauf indication expresse différente, tous les pourcentages (%) indiqués sont des % en masse. L'abréviation "pce" signifie parties en poids pour cent parties d'élastomère (du total des élastomères si plusieurs élastomères sont présents).

D'autre part, tout intervalle de valeurs désigné par l'expression "entre a et b" représente le domaine de valeurs supérieur à "a" et inférieur à "b" (c'est-à-dire bornes a et b exclues) tandis que tout intervalle de valeurs désigné par l'expression "de a à b" signifie le domaine de valeurs allant de "a" jusqu'à "b" (c'est-à-dire incluant les bornes strictes a et b).

Les composés mentionnés dans la description peuvent être d'origine fossile ou biosourcés. Dans ce dernier cas, ils peuvent être, partiellement ou totalement, issus de la biomasse ou obtenus à partir de matières premières renouvelables issues de la biomasse. Sont aussi concernés les polymères, les plastifiants, les charges....

Le terme composé 1,3-dipolaire est compris selon la définition donnée par IUPAC. Par définition, il contient un dipôle.

Dans la présente demande, on entend par groupe carboné un groupe qui contient un ou plusieurs atomes de carbone. On entend aussi par groupe d'atomes un groupe constitué par l'enchaînement de plusieurs atomes liés de façon covalente.

Dans la présente demande, un atome de carbone qui est membre du cycle époxyde est qualifié de carbone tertiaire lorsque l'atome de carbone est relié directement de façon covalente à deux atomes de carbone dont un est aussi membre du cycle époxyde et à l'atome d'oxygène membre du cycle époxyde. Un atome de carbone qui est membre du cycle époxyde est qualifié de carbone quaternaire lorsque l'atome de carbone est relié directement de façon covalente à trois atomes de carbone dont un est aussi membre du cycle époxyde et à l'atome d'oxygène membre du cycle époxyde.

Le composé 1,3 dipolaire est un oxyde de nitrile aromatique, c'est-à-dire un composé aromatique substitué par un dipôle oxyde de nitrile (-C≡N→O). Plus précisément, le composé 1,3-dipolaire est un monooxyde de nitrile aromatique, ce qui correspond à un composé qui contient un seul dipôle oxyde de nitrile et qui est un composé aromatique substitué par le dipôle oxyde de nitrile.

Le composé 1,3-dipolaire a pour caractéristique essentielle de comporter en plus du dipôle un groupe époxyde. De manière connue, un groupe époxyde est un cycle éther à 3 membres dont 2 membres sont deux atomes de carbone et le troisième membre est un atome d'oxygène. Le groupe époxyde utile aux besoins de l'invention est un cycle éther à 3 membres dont un premier membre est un atome de carbone présentant un rattachement au dipôle et un deuxième membre est un carbone tertiaire ou quaternaire. De préférence, le groupe époxyde est de formule (I).

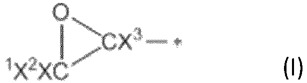

Dans la formule (I), laquelle le symbole ^{∗} représente un rattachement au dipôle ; les symboles X¹, X² et X³, identiques ou différents, représentent un atome d'hydrogène ou un groupe substituant, et au moins un des symboles X¹, X² et X³ est différent d'un atome d'hydrogène.

Selon un mode de réalisation préférentiel, X³ représente un atome d'hydrogène, ce qui revient à dire que le premier membre du cycle époxyde est un carbone tertiaire.

Selon un autre mode de réalisation préférentiel, X¹ représente un groupe substituant et X² représente un atome d'hydrogène, auquel cas le deuxième membre du cycle époxyde est un carbone tertiaire.

Selon un autre mode de réalisation préférentiel de l'invention, X¹ et X² représentent chacun un groupe substituant, auquel cas le deuxième membre du cycle époxyde est un carbone quaternaire.

De préférence, le groupe substituant, représenté par les symboles X¹ X² ou X³, est un groupe carboné, en particulier hydrocarboné. Le groupe substituant peut être aliphatique ou aromatique, linéaire, ramifié ou cyclique. Comme groupe substituant conviennent particulièrement les alkyles et les aryles, plus particulièrement les alkyles ayant 1 à 6 atomes de carbone, de préférence méthyle, ou les aryles ayant 6 à 12 atomes de carbone, de préférence phényle.

Selon un mode de réalisation, le composé 1,3-dipolaire comprend un noyau benzénique substitué par le dipôle du composé 1,3-dipolaire et de préférence aussi substitué en ortho du dipôle.

Selon un mode de réalisation très particulier de l'invention, le composé 1,3-dipolaire contient un motif de formule (II) dans laquelle quatre des cinq symboles R1 à R5, identiques ou différents, sont chacun un atome ou un groupe d'atomes, et le cinquième symbole représente une chaîne carbonée permettant le rattachement au groupe époxyde, sachant qu'au moins un des R1 et R5 est différent d'un atome d'hydrogène.

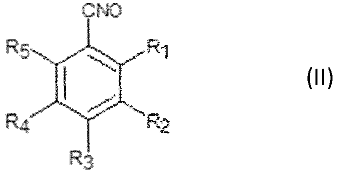

On entend par groupe d'atomes un enchaînement d'atomes liés de façon covalente pour former une chaîne. Deux groupes Ri et Ri+1, pour i nombre entier allant 1 à 4, peuvent former ensemble avec les atomes de carbone du noyau benzénique auxquels ils se rattachent, un cycle.

De préférence R1, R3 et R5 représentent chacun un groupe hydrocarboné et R2 ou R4 représente le cinquième symbole. De manière plus préférentielle R1, R3 et R5 représentent chacun un alkyle, de manière encore plus préférentielle un méthyle ou un éthyle.

La chaîne carbonée représentée par le cinquième symbole peut être aliphatique ou aromatique, linéaire, ramifiée ou cyclique, de préférence saturée. Le cinquième symbole représente préférentiellement une chaîne carbonée interrompue par un ou plusieurs hétéroatomes, de préférence oxygène. On entend par chaîne carbonée une chaîne qui comprend un ou plusieurs atomes de carbone. La chaîne carbonée peut être une chaîne hydrocarbonée. La chaîne carbonée peut comprendre une ou plusieurs fonctions éther, en particulier le cinquième symbole comprend un motif -CH₂O-, le groupe méthylène étant attaché au groupe époxyde.

Très avantageusement, le composé 1,3-dipolaire est un composé de formule (III), (IV) ou (V).

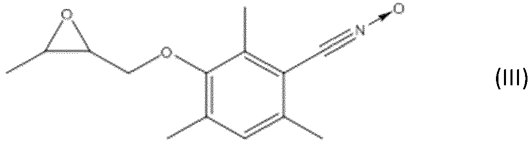

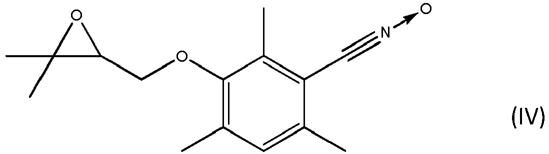

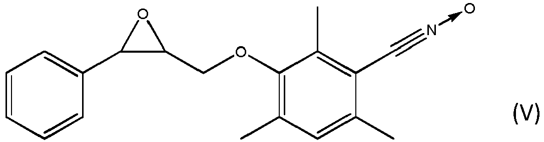

Le composé 1,3-dipolaire conforme à l'invention peut être utilisé pour greffer un ou plusieurs polymères diéniques, notamment élastomères, présents dans une composition de caoutchouc. Il peut être utilisé pour greffer un ou plusieurs groupes époxyde sur le polymère, de préférence plusieurs groupes. Le taux de composé 1,3-dipolaire peut varier dans une large mesure selon l'application envisagée de la composition de caoutchouc. Selon l'un quelconque des modes de réalisation de l'invention, le composé 1,3-dipolaire est préférentiellement introduit dans la composition de caoutchouc à un taux allant de 0,01 à 5% molaire, plus préférentiellement de 0,01 à 1% molaire, encore plus préférentiellement de 0,1 à 1% molaire. Ce taux exprimé en pourcentage molaire, équivaut au nombre de moles de composé 1,3-dipolaire pour 100 moles d'unités monomères diéniques de polymère diénique à greffer. On entend par unité monomère diénique toute unité qui résulte de l'insertion d'un diène dans une chaîne polymère et qui contient une double liaison carbone-carbone.

Le greffage procède d'une réaction de cycloaddition [2+3] du dipôle sur une double liaison carbone-carbone selon un mécanisme bien connu, la réaction pouvant être conduite en masse ou en solution. Lorsque le greffage est réalisé en masse, il est réalisé préférentiellement en présence d'un antioxydant. Le greffage en masse est mis en œuvre par exemple dans un mélangeur interne ou un mélangeur externe tel qu'un mélangeur à cylindres, soit à une température du mélangeur externe ou du mélangeur interne inférieure à 60°C, suivi d'une étape de réaction de greffage sous presse ou en étuve à des températures allant de 80°C à 200°C, soit à une température du mélangeur externe ou du mélangeur interne supérieure à 60°C sans traitement thermique postérieur. Le greffage du composé 1,3-dipolaire sur le polymère peut être réalisé préalablement à l'introduction du polymère dans la composition de caoutchouc, ou au cours de la fabrication de la composition.

Par polymère diénique, doit être compris un polymère comprenant des unités monomères diéniques qui présentent une double liaison carbone-carbone, en particulier des unités de monomères 1,3-diènes.

Le polymère diénique de départ susceptible d'être modifié par le procédé conforme à l'invention peut être :
(a) - tout homopolymère d'un monomère diène conjugué, notamment tout homopolymère obtenu par polymérisation d'un monomère diène conjugué ayant de 4 à 12 atomes de carbone ;
(b) - tout copolymère obtenu par copolymérisation d'un ou plusieurs diènes conjugués entre eux ou avec un ou plusieurs composés vinyle aromatique ayant de 8 à 20 atomes de carbone ;
(c) - un copolymère ternaire obtenu par copolymérisation d'éthylène, d'une α-oléfine ayant 3 à 6 atomes de carbone avec un monomère diène non conjugué ayant de 6 à 12 atomes de carbone, comme par exemple les élastomères obtenus à partir d'éthylène, de propylène avec un monomère diène non conjugué du type précité tel que notamment l'hexadiène-1,4, l'éthylidène norbornène, le dicyclopentadiène ;
(d) - un copolymère d'isobutène et d'isoprène (caoutchouc butyle), ainsi que les versions halogénées, en particulier chlorées ou bromées, de ce type de copolymère ;
(e) - tout copolymère obtenu par copolymérisation d'un ou plusieurs diènes conjugués avec l'éthylène, une α-monooléfine aliphatique acyclique ayant 3 à 18 atomes de carbone ou leur mélange comme par exemple ceux décrits dans le document WO 2005028526, WO 2004035639 et WO 2007054224.

De préférence, le polymère diénique de départ est un homopolymère de 1,3-butadiène, un homopolymère d'isoprène, un copolymère de 1,3-butadiène, un copolymère d'isoprène ou leurs mélanges. Le polymère diénique de départ est de manière plus préférentielle un élastomère diénique, notamment un homopolymère de 1,3-butadiène, un homopolymère d'isoprène, un copolymère de 1,3-butadiène, un copolymère d'isoprène ou leurs mélanges.

Le polymère modifié conforme à l'invention qui peut être obtenu selon le procédé conforme à l'invention, est de préférence un élastomère diénique, c'est-à-dire un élastomère qui contient à la fois des unités diéniques et des groupes époxydes décrits selon l'un quelconque des modes de réalisation de l'invention. L'élastomère modifié peut être utilisé dans une composition de caoutchouc, notamment pour pneumatique. Dans ce cas, la composition de caoutchouc contient, en plus de l'élastomère modifié, une charge renforçante et un système de réticulation. La composition de caoutchouc peut être à l'état cru (avant réticulation) ou à l'état cuit (après réticulation).

La composition de caoutchouc comporte tout type de charge dite renforçante, connue pour ses capacités à renforcer une composition de caoutchouc utilisable pour la fabrication de pneumatiques, par exemple une charge organique telle que du noir de carbone, une charge inorganique renforçante telle que de la silice à laquelle est associé de manière connue un agent de couplage, ou encore un mélange de ces deux types de charge. Une telle charge renforçante consiste typiquement en des nanoparticules dont la taille moyenne (en masse) est inférieure au micromètre, généralement inférieure à 500 nm, le plus souvent comprise entre 20 et 200 nm, en particulier et plus préférentiellement comprise entre 20 et 150 nm.

Selon un mode de réalisation particulier de l'invention, la charge renforçante comprend une charge inorganique, préférentiellement une silice. Selon ce mode de réalisation, la charge inorganique renforçante représente plus de 50% en masse de la masse de la charge renforçante de la composition de caoutchouc. On dit alors que la charge inorganique renforçante est majoritaire.

Lorsqu'il est combiné à une charge inorganique renforçante majoritaire telle que la silice, le noir de carbone est utilisé de préférence à un taux inférieur à 20 pce, plus préférentiellement inférieur à 10 pce (par exemple entre 0.5 et 20 pce, notamment entre 2 et 10 pce). Dans les intervalles indiqués, on bénéficie des propriétés colorantes (agent de pigmentation noire) et anti-UV des noirs de carbone, sans pénaliser par ailleurs les performances typiques apportées par la charge inorganique renforçante.

De manière préférentielle, le taux de charge renforçante totale est compris entre 30 et 160 pce, plus préférentiellement entre 40 pce et 160 pce. En deçà de 30 pce, le renforcement de la composition de caoutchouc est insuffisant pour apporter un niveau de cohésion ou de résistance à l'usure adéquats du composant caoutchouteux du pneumatique comprenant cette composition. De manière encore plus préférentielle, le taux de charge renforçante totale est d'au moins 50 pce. Au-delà de 160 pce, il existe un risque d'augmentation de l'hystérèse et donc de la résistance au roulement des pneumatiques. Pour cette raison, le taux de charge renforçante totale est de préférence dans un domaine allant de 50 à 120 pce, notamment pour un usage dans une bande de roulement de pneumatique. L'une quelconque de ces plages de taux de charge renforçante totale peut s'appliquer à l'un quelconque des modes de réalisation de l'invention.

Pour coupler la charge inorganique renforçante à l'élastomère diénique, on utilise de manière bien connue un agent de couplage, notamment un silane, (ou agent de liaison) au moins bifonctionnel destiné à assurer une connexion suffisante, de nature chimique et/ou physique, entre la charge inorganique (surface de ses particules) et l'élastomère diénique. On utilise en particulier des organosilanes ou des polyorganosiloxanes au moins bifonctionnels. Plus particulièrement, on utilise des silanes polysulfurés, dits "symétriques" ou "asymétriques" selon leur structure particulière, tels que décrits par exemple dans les demandes WO03/002648 (ou US 2005/016651) et WO03/002649 (ou US 2005/016650). A titre d'exemples de silanes polysulfurés, on citera plus particulièrement les polysulfures (notamment disulfures, trisulfures ou tétrasulfures) de bis-(alkoxyl(C₁-C₄)-alkyl(C₁-C₄)silyl-alkyl(C₁-C₄)), comme par exemple les polysulfures de bis(3-triméthoxysilylpropyl) ou de bis(3-triéthoxysilylpropyl). Parmi ces composés, on utilise en particulier le tétrasulfure de bis(3-triéthoxysilylpropyl), en abrégé TESPT, de formule [(C₂H₅O)₃Si(CH₂)₃S₂]₂ ou le disulfure de bis-(triéthoxysilylpropyle), en abrégé TESPD, de formule [(C₂H₅O)₃Si(CH₂)₃S]₂.

La teneur en agent de couplage est avantageusement inférieure à 20 pce, étant entendu qu'il est en général souhaitable d'en utiliser le moins possible. Typiquement le taux d'agent de couplage représente de 0,5% à 15% en poids par rapport à la quantité de charge inorganique.

Son taux est préférentiellement compris entre 0,5 et 12 pce, plus préférentiellement compris dans un domaine allant de 3 à 10 pce. Ce taux est aisément ajusté par l'homme du métier selon le taux de charge inorganique utilisé dans la composition.

La composition de caoutchouc conforme à l'invention peut également contenir, en complément des agents de couplage, des activateurs de couplage, des agents de recouvrement des charges inorganiques ou plus généralement des agents d'aide à la mise en œuvre susceptibles de manière connue, grâce à une amélioration de la dispersion de la charge dans la matrice de caoutchouc et à un abaissement de la viscosité des compositions, d'améliorer leur faculté de mise en œuvre à l'état cru.

La composition de caoutchouc conforme à l'invention peut comporter également tout ou partie des additifs usuels habituellement utilisés dans les compositions d'élastomères destinées à constituer des mélanges externes d'articles finis en caoutchouc tels que des pneumatiques, en particulier de bandes de roulement, comme par exemple des plastifiants ou des huiles d'extension, des pigments, des agents de protection tels que cires anti-ozone, anti-ozonants chimiques, anti-oxydants, des agents anti-fatigue, des résines renforçantes (tels que résorcinol ou bismaléimide), des accepteurs (par exemple résine phénolique novolaque) ou des donneurs de méthylène (par exemple HMT ou H3M) tels que décrits par exemple dans la demande WO 02/10269, un système de réticulation, des accélérateurs ou retardateurs de vulcanisation, des activateurs de vulcanisation. Le système de réticulation est de préférence à base de soufre, mais il peut être à base de polyacides, notamment diacides comme décrits dans les demandes de brevet WO2014095582 et WO2014095585.

La composition de caoutchouc conforme à l'invention est fabriquée dans des mélangeurs appropriés, en utilisant deux phases de préparation successives bien connues de l'homme du métier : une première phase de travail ou malaxage thermomécanique (phase dite « non-productive ») à haute température, jusqu'à une température maximale comprise entre 130°C et 200°C, suivie d'une seconde phase de travail mécanique (phase dite « productive ») jusqu'à une plus basse température, typiquement inférieure à 110°C, par exemple entre 40°C et 100°C, phase de finition au cours de laquelle est incorporé le système de réticulation.

La composition de caoutchouc conforme à l'invention peut être soit à l'état cru (avant réticulation ou vulcanisation), soit à l'état cuit (après réticulation ou vulcanisation). Elle est préférentiellement utilisée dans un pneumatique, par exemple comme article semi-fini, en particulier une bande de roulement.

Les caractéristiques précitées de la présente invention, ainsi que d'autres, seront mieux comprises à la lecture de la description suivante de plusieurs exemples de réalisation de l'invention, donnés à titre illustratif et non limitatif.

### II. EXEMPLES DE REALISATION DE L'INVENTION

### 11.1-Mesures et tests utilisés :

### Analyse RMN :

L'analyse structurale ainsi que la détermination des puretés molaires des molécules de synthèses sont réalisées par une analyse RMN. Les spectres sont acquis sur un spectromètre Avance 3 400 MHz BRUKER équipé d'une sonde " large bande " BBFO-zgrad 5 mm. L'expérience RMN ¹H quantitative, utilise une séquence simple impulsion 30° et un délai de répétition de 3 secondes entre chacune des 64 acquisitions. Les échantillons sont solubilisés dans le Diméthylsulfoxide deutéré (DMSO). Ce solvant est également utilisé pour le signal de lock. La calibration est réalisée sur le signal des protons du DMSO deutéré à 2.44ppm par rapport à une référence TMS à 0ppm. Le spectre RMN ¹H couplé aux expériences 2D HSQC ¹H/13C et HMBC ¹H/¹³C permettent la détermination structurale des molécules (cf tableaux d'attributions). Les quantifications molaires sont réalisées à partir du spectre RMN 1D ¹H quantitatif.

La détermination du taux molaire de composé oxyde de nitrile greffé est réalisée par une analyse RMN. Les spectres sont acquis sur un spectromètre 500 MHz BRUKER équipé d'une « CryoSonde BBFO-zgrad-5 mm ». L'expérience RMN ¹H quantitative, utilise une séquence simple impulsion 30° et un délai de répétition de 5 secondes entre chaque acquisition. Les échantillons sont solubilisés dans le chloroforme deutéré (CDCl3) dans le but d'obtenir un signal de « lock ».

Des expériences RMN 2D ont permis de vérifier la nature du motif greffé grâce aux déplacements chimiques des atomes de carbone et de proton.

### Essais de traction :

Les allongements à la rupture et les contraintes à la rupture sont mesurés par des essais de traction selon la norme française NF T 46-002 de septembre 1988. Toutes ces mesures de traction sont effectuées dans les conditions normales de température (23 ± 30 2°C) et d'hygrométrie (50 ± 5% d'humidité relative), selon la norme française NF T 40-101 (décembre 1979).

### Propriétés dynamiques :

Les propriétés dynamiques tan(δ)max sont mesurées sur un viscoanalyseur (Metravib VA4000), selon la norme ASTM D 5992-96. On enregistre la réponse d'un échantillon de composition vulcanisée (éprouvette cylindrique de 4 mm d'épaisseur et de 400 mm² de section), soumis à une sollicitation sinusoïdale en cisaillement simple alterné, à la fréquence de 10Hz, dans les conditions normales de température (23°C) selon la norme ASTM D 1349-99. On effectue un balayage en amplitude de déformation de 0,1% à 100% (cycle aller), puis de 100% à 0,1% (cycle retour). Les résultats exploités sont le module complexe de cisaillement dynamique (G^{∗}) à 25% de déformation, le facteur de perte tan(δ) et l'écart de module (ΔG^{∗}) entre les valeurs à 0,1 et 100% de déformation (effet Payne). Pour le cycle retour, on indique la valeur maximale de tan(δ) observée, noté tan(δ)max.

### Rhéometrie :

Les mesures sont effectuées à 150°C avec un rhéomètre à chambre oscillante, selon la norme DIN 53529 - partie 3 (juin 1983). Les mesures sont traitées selon la norme DIN 53529 - partie 2 (mars 1983). L'évolution du couple rhéométrique en fonction du temps décrit l'évolution de la rigidification de la composition par suite de la réaction de vulcanisation et permet ainsi de suivre l'avancement de la vulcanisation. On mesure pour chaque composition la valeur de couple minimum Cmin. Le Cmin est représentatif de la viscosité à cru (avant vulcanisation) de la composition de caoutchouc et permet d'appréhender l'aptitude de la composition de caoutchouc à être mise en œuvre (en anglais « processability »).

### II.2-Synthèse des composés 1,3-dipolaires :

Les composés 1,3-dipolaires suivants ont été préparés, respectivement D-1, D-2, D-3 et D-4.

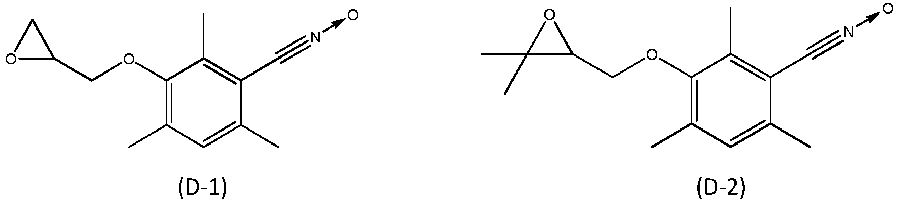

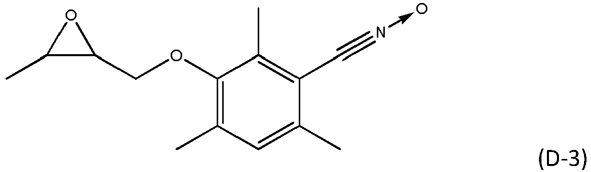

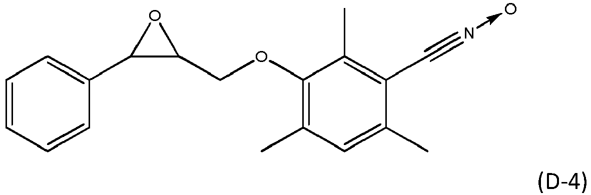

### Synthèse du 3-hydroxy-2,4,6-triméthylbenzaldéhyde (cible 1) :

Le composé cible 1 (ou A) est un précurseur commun utilisé dans la synthèse de certains des composés 1,3-dipolaires. Il est synthétisé selon le schéma suivant :

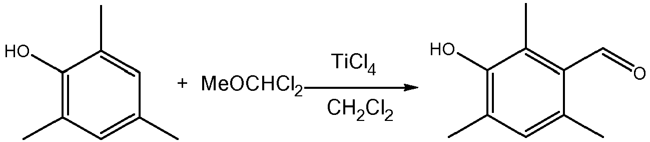

Le composé cible 1 peut être obtenu selon une procédure décrite dans l'article Yakubov, A. P.; Tsyganov, D. V.; Belen'kii, L. I.; Krayushkin, M. M. Bulletin of the Academy of Sciences of the USSR, Division of Chemical Science (English Translation); vol. 40; nb. 7.2; (1991); p. 1427 - 1432*;* Izvestiya Akademii Nauk SSSR, Seriya Khimicheskaya; nb. 7; (1991); p. 1609 -1615*.*

### Synthèse du 2,4,6-triméthyl-3-(oxiran-2-ylméthoxy)benzonitrile oxyde (D-1):

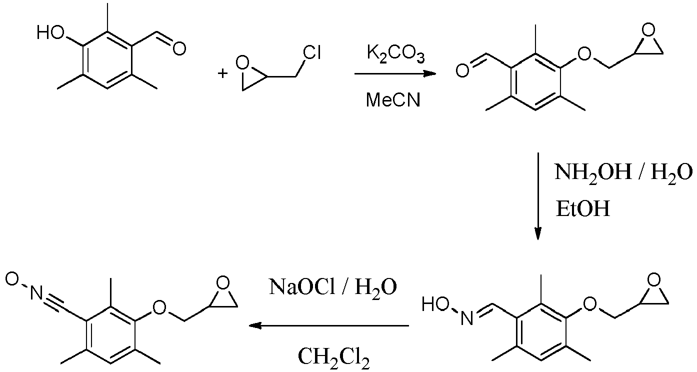

### Synthèse de 2,4,6-triméthyl-3-(oxiran-2-ylméthoxy)benzaldéhyde (cible 2) :

A un mélange de 3-hydroxy-2,4,6-triméthylbenzaldéhyde (40,00 g, 0,244 mol) et d'épichlorhydrine (56,35 g, 0,609 mol) dans l'acétonitrile (100 ml) est ajouté le carbonate de potassium (50,50 g, 0,365 mol). Le milieu réactionnel est agité pendant 3 heures à 60 ºC et ensuite agité pendant 2.5-3 heures à 70 ºC. Après retour à 40-50 °C, le mélange réactionnel est dilué avec un mélange d'eau (250ml) et d'éthylacétate (250 ml), puis maintenu sous agitation pendant 10 minutes. La phase organique est séparée et lavée avec de l'eau (4 fois par 125 ml). Le solvant est évaporé sous pression réduite (T_{bain} 37 ºC, 40 mbar). Une huile rouge (66,43 g) est obtenue.

Le produit secondaire de la réaction, le 3,3'-((2-hydroxypropane-1,3-diyl)bis(oxy))bis(2,4,6-triméthylbenzaldéhyde) est séparé du produit cible 2 par chromatographie sur colonne de silice (éluent : éthylacétate / éther de pétrole = 1 / 4). Après récupération des fractions du produit cible 2, les solvants sont évaporés sous pression réduite (Tbain 36 ºC, 21 mbar). De l'éther de pétrole (120 ml) est ajouté au résidu et la suspension est maintenue sous agitation à -18ºC pendant 2 heures. Le précipité est filtré et lavé sur le filtre avec de l'éther de pétrole (40/60) (3 fois 25 ml) et enfin séché pendant 10-15 heures sous pression atmosphérique à température ambiante. Un solide blanc (40,04 g, rendement massique de 75 %) avec un point de fusion de 52 °C est obtenu. La pureté molaire est supérieure à 99 % (RMN ¹H).

**Tableau d'attribution :**

| 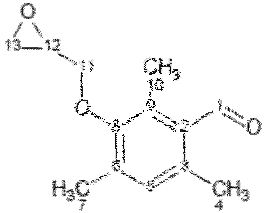 | | |
|---|---|---|
| | δ ¹H (ppm) | δ ¹³C (ppm) |
| 1 | 10.37 | 193.3 |
| 2 | / | 131.1 |
| 3 | / | 132.8 |
| 4 | 2.4 | 19.2 |
| 5 | 6.94 | 131.3 |
| 6 | / | 136.3 |
| 7 | 2.2 | 16.1 |
| 8 | / | 153.4 |
| 9 | / | 135.7 |
| 10 | 2.4 | 11.7 |
| 11 | 3.50/4.00 | 73.4 |
| 12 | 3.29 | 49.6 |
| 13 | 2.60/2.76 | 42.9 |

### Solvant DMSO

### Synthèse de 2,4,6-triméthyl-3-(oxiran-2-ylmethoxy)benzaldéhyde oxime (cible 3) :

A une solution de 2,4,6-triméthyl-3-(oxiran-2-ylméthoxy)benzaldéhyde (46,70 g, 0,212 mol) dans l'alcool éthylique (750 ml) est ajoutée à température ambiante une solution d'hydroxylamine (16,81 g, 0,254 mol, 50 % dans l'eau, Aldrich) dans l'alcool éthylique (75 ml). Le milieu réactionnel est agité pendant 3 heures à 23°C (T_{bain}). Après évaporation du solvant (T_{bain} 24°C, 35 mbar), de l'éther de pétrole (40/60) (150 ml) est ajouté. Le précipité est filtré et lavé sur le filtre par l'éther de pétrole (100 ml). Le produit brut est solubilisé dans un mélange d'acétate d'éthyle (650 ml) et d'éther de pétrole (650 ml) à température ambiante et cette solution est filtrée sur une couche de silicagel (Ø9 cm, 2,0 cm de SiO₂).

Les solvants sont évaporés (T_{bain} 22-24°C) et le produit cible 3 est séché sous pression atmosphérique à température ambiante. Un solide blanc (43,81 g, rendement massique de 88 %) avec un point de fusion de 77 °C est obtenu. La pureté molaire est supérieure à 99 % (RMN ¹H).

**Tableau d'attribution :**

| 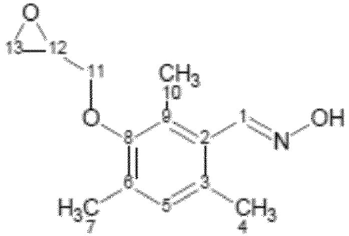 | | |
|---|---|---|
| | δ ¹H (ppm) | δ ¹³C (ppm) |
| 1 | 8.2 | 147.3 |
| 2 | / | 129.1 |
| 3 | / | 129.2 |
| 4 | 2.18 | 20.1 |
| 5 | 6.85 | 130.2 |
| 6 | / | 130.3 |
| 7 | 2.15 | 15.7 |
| 8 | / | 153.1 |
| 9 | / | 131.7 |
| 10 | 2.18 | 13.1 |
| 11 | 3.48/3.96 | 73.3 |
| 12 | 3.27 | 49.6 |
| 13 | 2.60/2.76 | 42.8 |

### Solvant DMSO

### Synthèse de 2,4,6-triméthyl-3-(oxiran-2-ylméthoxy)benzonitrile oxyde (D-1) :

A une solution de 2,4,6-triméthyl-3-(oxiran-2-ylméthoxy)benzaldéhyde oxime (17,00 g, 0,072 mol) dans du dichlorométhane (350 ml) refroidie jusqu'à 3°C est ajoutée au goutte à goutte une solution aqueuse de NaOCl dans l'eau (62,9 g Cl actif/L) (126 ml) pendant 10-15 minutes. La température du milieu réactionnel reste comprise entre 3 et 5°C. Le milieu réactionnel est ensuite agité pendant 1 heure à température 3-5°C. La phase aqueuse est séparée et extraite avec du dichlorométhane (25 ml). Les phases organiques réunies sont lavées avec de l'eau (3 fois 75 ml). Le solvant est évaporé à pression réduite (T_{bain} 22°C, 35 mbar). A ce résidu est additionné de l'éther de pétrole (40/60) (90 ml) et la suspension est maintenue sous agitation à température ambiante pendant 10-12 heures. Le précipité est filtré et lavé sur le filtre avec de l'éther de pétrole (3 fois par 30 ml) et enfin séché pendant 10-15 heures sous pression atmosphérique à température ambiante. Un solide blanc (15,12 g, rendement massique de 90 %) avec un point de fusion de 63°C est obtenu. La pureté molaire est supérieure à 99 % (RMN ¹H).

**Tableau d'attribution :**

| 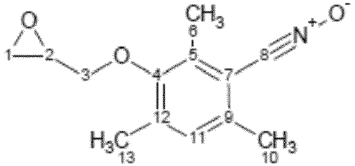 | | |
|---|---|---|
| | δ ¹H (ppm) | δ ¹³C (ppm) |
| 1 | 2.59/2.76 | 43.0 |
| 2 | 3.28 | 49.6 |
| 3 | 3.51/4.03 | 73.5 |
| 4 | / | 153.0 |
| 5 | / | 136.3 |
| 6 | 2.27 | 14.3 |
| 7 | / | 111.7 |
| 8 | / | / |
| 9 | / | 134.4 |
| 10 | 2.18 | 15.9 |
| 11 | 7.01 | 129.9 |
| 12 | / | 134.0 |
| 13 | 2.27 | 19.5 |

### Solvant DMSO

### Synthèse de 2,4,6-triméthyl-3-(3-(3,3-diméthyloxiran-2-yl)propoxy)benzonitrile oxyde (D-2) :

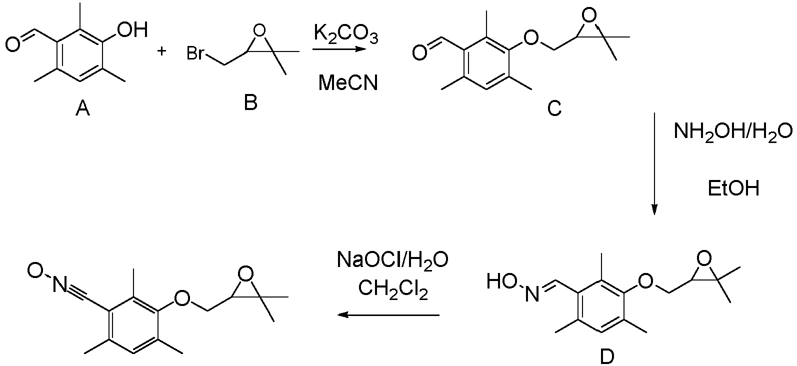

### Synthèse de 3-(bromométhyl)-2,2-diméthyloxirane (B) :

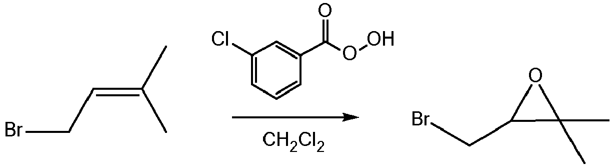

Le composé B peut être obtenu selon une procédure décrite dans l'article Shimizu, Hitoshi et al; Organic Process Research &Development, 9(3), 278-287; 2005*.*

### Synthèse de 3-((3,3-diméthyloxiran-2-yl)méthoxy)-2,4,6-triméthylbenzaldéhyde (C) :

A un mélange de 3-hydroxy-2,4,6-triméthylbenzaldéhyde (19,20 g, 0,117 mol) et de 3-(bromométhyl)-2,2-diméthyloxirane (19,30 g, 0,117 mol) dans l'acétonitrile (50 ml) est ajouté le carbonate de potassium (12,12 g, 0,877 mol). Le milieu réactionnel est agité pendant 10-11 heures à 60 ºC (T_{bain}). Après retour à température ambiante, le mélange réactionnel est dilué avec un mélange d'eau (700 ml) et d'acétate d'éthyle (100 ml) et agité pendant 10 minutes. La phase aqueuse est séparée et extraite avec de l'acétate d'éthyle (3 fois 75 ml). Les phases organiques réunies sont lavées 2 fois avec une solution de NaOH (8,0 g dans 100 ml d'eau) et de l'eau (5 fois 75 ml). Le solvant est évaporé sous pression réduite (T_{bain} 35 ºC, 10 mbar). Une huile jaune claire (28,18 g, rendement massique de 97 %) est obtenue. La pureté molaire est supérieure à 85 % (RMN ¹H). Le produit C est utilisé pour l'étape suivante sans aucune purification supplémentaire.

**Tableau d'attribution :**

| 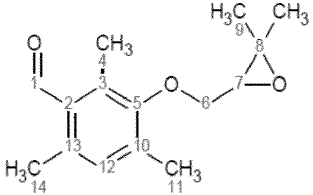 | | |
|---|---|---|
| | δ ¹H (ppm) | δ ¹³C (ppm) |
| 1 | 10.4 | 192.6 |
| 2 | / | 131.2 |
| 3 | / | 133.3 |
| 4 | 2.43 | 12 |
| 5 | / | 153.7 |
| 6 | 3.67 et3.87 | 71.3 |
| 7 | 3.09 | 61.1 |
| 8 | / | 57.8 |
| 9 | 1.17 et 1.28 | 18.6 et 24.3 |
| 10 | / | 125.8 |
| 11 | 2.21 | 16.5 |
| 12 | 6.79 | 13.5 |
| 13 | / | 136.4 |
| 14 | 2.4 | 19.5 |

### Solvant CDCl₃

### Synthèse de 3-((3,3-diméthyloxiran-2-yl)méthoxy)-2,4,6-triméthylbenzaldéhyde oxime (D) :

A une solution de 3-((3,3-diméthyloxiran-2-yl)méthoxy)-2,4,6-triméthylbenzaldéhyde (11,8 g, 0,475 mol) dans l'alcool éthylique (25 ml) est ajoutée à 40°C (T_{bain}) une solution d'hydroxylamine (5,02 g, 0,760 mol, 50 % dans l'eau, Aldrich) dans l'alcool éthylique (10 ml). Le milieu réactionnel est agité pendant 2,5-3,0 heures à température 55°C (T_{bain}). Après évaporation du solvant (T_{bain} 32°C, 26 mbar), un mélange d'acétate d'éthyle (20 ml), d'éther de pétrole (40/60) (30 ml) et d'eau (10 ml) est ajouté.

Ensuite, la phase organique est séparée et lavée avec de l'eau (10 ml). La solution est filtrée sur une couche de silicagel (Ø 3,5 cm, h = 2,0 cm), puis la couche de silicagel est lavée par un mélange d'acétate d'éthyle (10 ml) et d'éther de pétrole (20 ml). Après évaporation des solvants (T_{bain} 33°C, 11 mbar) une huile incolore (10,33 g, rendement massique de 83%) est obtenue. La pureté molaire est supérieure à 78 % (RMN ¹H) et 16% d'EtOAc. Le produit D est utilisé dans l'étape suivante sans séchage supplémentaire.

**Tableau d'attribution :**

| 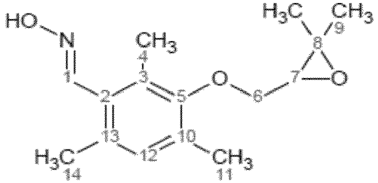 | | |
|---|---|---|
| | δ¹H (ppm) | δ ¹³C (ppm) |
| 1 | 8.29 | 149.2 |
| 2 | / | 128.3 |
| 3 | / | 129.9 |
| 4 | 2.27 | 13.3 |
| 5 | / | 153.5 |
| 6 | 3.76 et 3.88 | 71.2 |
| 7 | 3.15 | 61.4 |
| 8 | / | 58 |
| 9 | 1.22 et 1.33 | 18.6 et 24.4 |
| 10 | / | 131.3 |
| 11 | 2.22 | 16.1 |
| 12 | 6.83 | 130.5 |
| 13 | / | 132.7 |
| 14 | 2.25 | 20.4 |

### Solvant CDCl₃

### Synthèse du 3-((3,3-diméthyloxiran-2-yl)méthoxy)-2,4,6-triméthylbenzonitrile oxyde (D-2) :

A une solution de 3-((3,3-diméthyloxiran-2-yl)méthoxy)-2,4,6-trimethylbenzaldehyde oxime (9,90 g, 0.367 mol) dans le dichlorométhane (350 ml) refroidie jusqu'à 1-3°C est ajoutée au goutte à goutte une solution aqueuse de NaOCl dans l'eau (62,9 g Cl/L) (65 ml) pendant 15 minutes. La température du milieu réactionnel reste comprise entre 2-3°C. Le milieu réactionnel est ensuite agité pendant 2 heures à 2-3°C. La phase organique est séparée et lavée avec de l'eau (3 fois 50 ml). Le solvant est évaporé à pression réduite (T_{bain} 21°C, 120 mbar). A ce résidu est additionné de l'éther de pétrole (40/60) (15 ml) et la suspension est maintenue à -18ºC pendant 2 heures. Le précipité est filtré et lavé sur le filtre par l'éther de pétrole (3 fois par 15 ml) et enfin séché pendant 10-15 heures sous pression atmosphérique à température ambiante. Un solide blanc (4,42 g, rendement massique de 45 %) avec un point de fusion de 84°C est obtenu. La pureté molaire est supérieure à 98 % (RMN ¹H).

**Tableau d'attribution :**

| 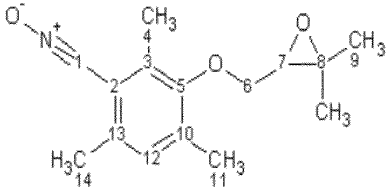 | | |
|---|---|---|
| | δ ¹H (ppm) | δ ¹³C (ppm) |
| 1 | / | / |
| 2 | / | 112.7 |
| 3 | / | 134.2 |
| 4 | 2.35 | 14.8 |
| 5 | / | 153.4 |
| 6 | 3.93/3.71 | 71.9 |
| 7 | 3.11 | 61.2 |
| 8 | / | 57.8 |
| 9 | 1.22 et 1.33 | 24.5/18.8 |
| 10 | / | 134.2 |
| 11 | 2.23 | 16.5 |
| 12 | 6.86 | 130.2 |
| 13 | / | 137.2 |
| 14 | 2.32 | 20 |

### Solvant CDCl₃

### Synthèse de 2,4,6-triméthyl-3-(3-(3-méthyloxiran-2-yl)propoxy)benzonitrile oxyde (D-3) :

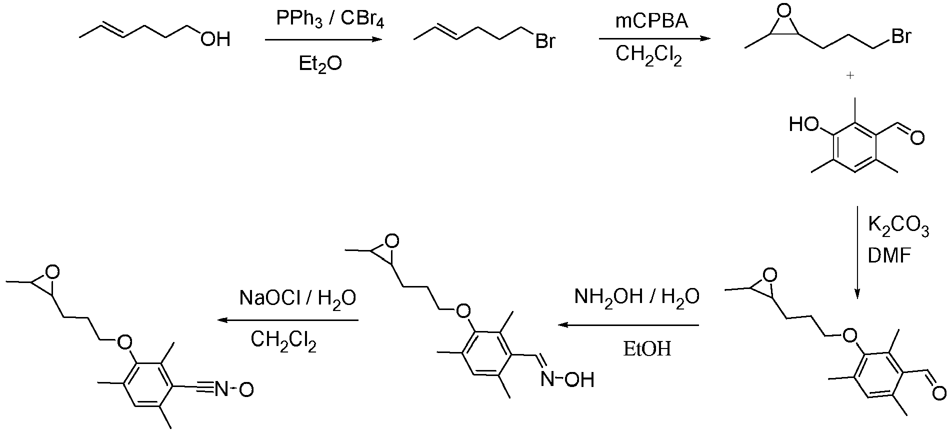

### Synthèse de 6-bromohex-2-ène :

Ce composé peut être obtenu par exemple selon une procédure décrite dans l'article Nicolai, Stefano et al. Tetrahedron, 71(35), 5959-5964; 2015*.*

### Synthèse de 2-(3-bromopropyl)-3-méthyloxirane :

Ce composé peut être obtenu par exemple selon une procédure décrite dans l'article Hu, Shanghai; Hager, Lowell P.; Tetrahedron Letters; vol. 40; nb. 9; (1999); p. 1641 -1644.

### Synthèse du 2,4,6-triméthyl-3-(3-(3-méthyloxiran-2-yl)propoxy)benzaldéhyde :

A un mélange de 3-hydroxy-2,4,6-triméthylbenzaldéhyde (10,00 g, 0,061 mol) et de 2-(3-bromopropyl)-3-méthyloxirane (10,39 g, 0,058 mol) dans le DMF (5 ml) est ajouté le carbonate de potassium (6,01 g, 0,044 mol). Le milieu réactionnel est agité pendant 1 heure à 80 ºC (T_{bain}) et 3 heures à 100 ºC (T_{bain}). Après retour à température ambiante, le mélange réactionnel est dilué avec un mélange d'eau (75 ml) et de chlorure de méthylène (50 ml). Le produit est extrait avec le chlorure de méthylène (2 fois 10 ml). Les phases organiques réunies sont lavées 2 fois avec une solution de NaOH (4 g dans 50 ml d'eau) et de l'eau (3 fois par 15 ml). Le solvant est évaporé sous pression réduite (T_{bain} 45 ºC, 8 mbar). Une huile (14,25 g, rendement massique de 93 %) est obtenue. La pureté molaire est supérieure à 85 % (RMN ¹H). Le produit est utilisé pour l'étape suivante sans purification supplémentaire.

**Tableau d'attribution :**

| 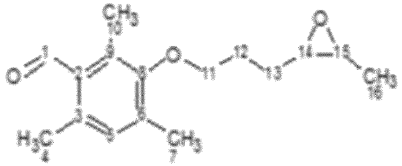 | | |
|---|---|---|
| | δ ¹H (ppm) | δ ¹³C (ppm) |
| 1 | 10.46 | 193.2 |
| 2 | / | 131.7 |
| 3 | / | 133.8 |
| 4 | 2.45 | 19.9 |
| 5 | 6.83 | 131.9 |
| 6 | / | 137 |
| 7 | 2.22 | 16.8 |
| 8 | / | 154.4 |
| 9 | / | 136.5 |
| 10 | 2.44 | 12.3 |
| 11 | 3.67 | 72.2 |
| 12 | 1.89 | 26.7 |
| 13 | 1.62-1.79 | 28.7 |
| 14 | 2.66 | 59.3 |
| 15 | 2.75 | 54.5 |
| 16 | 1.25 | 17.6 |

### Solvant CDCl₃

### Synthèse de 2,4,6-triméthyl-3-(3-(3-méthyloxiran-2-yl)propoxy)benzaldéhyde oxime :

A une solution de 2,4,6-triméthyl-3-(3-(3-methyloxiran-2-yl)propoxy)benzaldéhyde (14,00 g, 0,056 mol) dans l'alcool éthylique (40 ml) est ajoutée à 45°C une solution d'hydroxylamine (5,13 g, 0,078 mol, 50 % dans l'eau, Aldrich) dans l'alcool éthylique (10 ml). Le milieu réactionnel est agité pendant 1,5 heure à 50°C (T_{bain}). Après l'évaporation du solvant (T_{bain} 40°C, 45 mbar), du chlorure de méthylène (50 ml) est ajouté et la solution est lavée avec de l'eau (3 fois 15 ml). Ensuite, après évaporation du solvant (T_{bain} 40°C, 70 mbar), du chlorure de méthylène est ajouté. La suspension est agitée à température ambiante pendant 10 minutes et refroidie jusqu'à -18°C pendant 10-15 minutes. Le précipité est filtré et lavé sur le filtre 3 fois avec un mélange de chlorure de méthylène (1 ml) et d'éther de pétrole (4 ml) et enfin séché sous pression atmosphérique à température ambiante. Un solide blanc (10,02 g, rendement massique de 65 %) avec un point de fusion de 78°C est obtenu. La pureté molaire est supérieure à 90 % (RMN ¹H).

**Tableau d'attribution :**

| 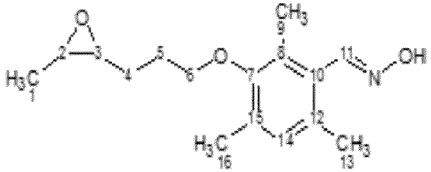 | | |
|---|---|---|
| | δ ¹H (ppm) | δ ¹³C (ppm) |
| 1 | 1.26 | 17.6 |
| 2 | 2.76 | 54.7 |
| 3 | 2.68 | 59.5 |
| 4 | 1.65/1.79 | 28.8 |
| 5 | 1.88 | 26.8 |
| 6 | 3.68 | 72.0 |
| 7 | / | 154.1 |
| 8 | / | 130.4 |
| 9 | 2.24 | 13.6 |
| 10 | / | 128.4 |
| 11 | 8.30 | 149.9 |
| 12 | / | 132.7 |
| 13 | 2.25 | 20.5 |
| 14 | 6.82 | 130.8 |
| 15 | / | 131.8 |
| 16 | 2.19 | 16.3 |

### Solvant CDCl₃

### Synthèse de 2,4,6-triméthyl-3-(3-(3-méthyloxiran-2-yl)propoxy)benzonitrile oxyde (D-3) :

A une solution de 2,4,6-triméthyl-3-(3-(3-méthyloxiran-2-yl)propoxy)benzaldéhyde oxime (3,35 g, 0,012 mol) dans le dichlorométhane (50 ml) refroidi jusqu'à 0°C (T_{bain}) est ajoutée au goutte à goutte une solution aqueuse de NaOCl dans l'eau (4% du chlore actif, Aldrich) (17 ml) pendant 5 minutes. La température du milieu réactionnel reste comprise entre 3 et 5°C. Le milieu réactionnel est ensuite agité pendant 1 heure à température 3-5°C. La phase aqueuse est séparée puis extraite avec du dichlorométhane (5 ml). Les phases organiques réunies sont lavées avec de l'eau (2 fois 5 ml). Le solvant est évaporé à pression réduite (T_{bain} 21°C, 16 mbar). A ce résidu est ajouté de l'éther de pétrole (40/60) (7 ml) et la suspension est agitée à température ambiante pendant 10 minutes. Le précipité est filtré et lavé sur le filtre par de l'éther de pétrole (2 fois 5 ml) et enfin séché sous pression atmosphérique à température ambiante. Un solide jaune clair (2,49 g, rendement massique de 75%) avec un point de fusion de 56°C est obtenu. La pureté molaire est supérieure à 94 % (RMN ¹H).

**Tableau d'attribution :**

| 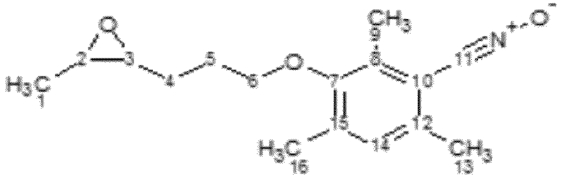 | | |
|---|---|---|
| | δ ¹H (ppm) | δ ¹³C (ppm) |
| 1 | 1.26 | 17.6 |
| 2 | 2.75 | 54.5 |
| 3 | 2.66 | 59.3 |
| 4 | 1.60/1.80 | 28.7 |
| 5 | 1.88 | 26.8 |
| 6 | 3.68 | 72.2 |
| 7 | / | 153.9 |
| 8 | / | 137.1 ou 134.6 |
| 9 | 2.31 | 14.9 |
| 10 | / | 112.8 |
| 11 | / | / |
| 12 | / | 137.1 ou 134.6 |
| 13 | 2.31 | 20.3 |
| 14 | 6.84 | 130.3 |
| 15 | / | 134.6 |
| 16 | 2.19 | 16.5 |

### Solvant CDCl₃

### Synthèse de 2,4,6-triméthyl-3-((3-phényloxiran-2-yl)méthoxy)benzonitrile oxyde (D-4) :

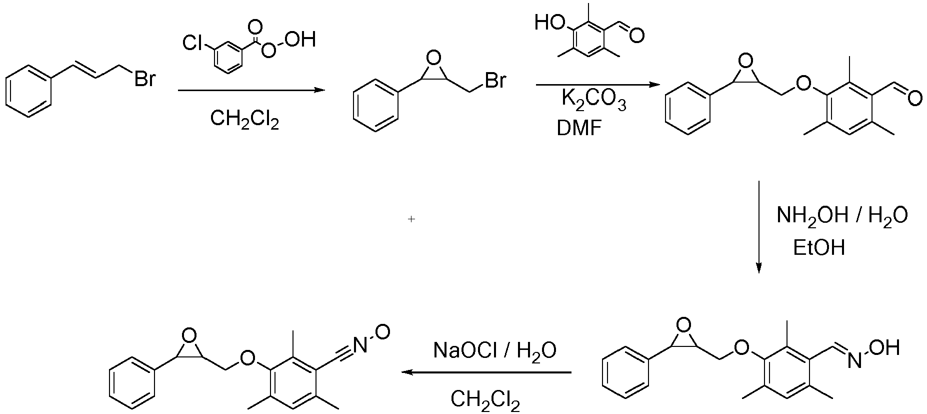

### Synthèse de 2-(bromométhyl)-3-phényloxirane :

Ce composé peut être obtenu selon une procédure décrite dans l'article Dickinson, Julia M. et al, Chemical Society, Perkin Transactions 1: Organic and Bio-Organic Chemistry (1972-1999), (4), 1179-84; 1990*.*

### Synthèse de 2,4,6-trimethyl-3-((3-phenyloxiran-2-yl)methoxy)benzaldehyde :

A un mélange de 3-hydroxy-2,4,6-triméthylbenzaldéhyde (13,50 g, 0,082 mol) et de 2-(bromométhyl)-3-phényloxirane (17,50 g, 0,082 mol) dans le DMF (8 ml) est ajouté le carbonate de potassium (8,51 g, 0,062 mol). Le milieu réactionnel est agité pendant 5-6 heures à 60 ºC (T_{bain}). Après retour à 40-50 ºC le mélange réactionnel est dilué avec un mélange d'eau (200 ml) et d'éthylacétate (70-80 ml). Le produit cible est extrait par avec de l'éthylacétate (2 fois 25 ml). Les phases organiques réunies sont lavées avec une solution de NaOH (8 g dans 70 ml d'eau) et d'eau (4 fois 25 ml). Le solvant est évaporé sous pression réduite (T_{bain} 34 ºC, 16 mbar). De l'éther de pétrole (40/60) (50 ml) est ajouté et le précipité est filtré et lavé sur le filtre avec un mélange d'éther de pétrole (15 ml) et d'éthylacétate (1 ml) et enfin séché sous pression atmosphérique à température ambiante.

Un solide beige (13,28 g, rendement massique de 55 %) avec un point de fusion de 53°C est obtenu. La pureté molaire est supérieure à 90 % (RMN ¹H).

**Tableau d'attribution :**

| 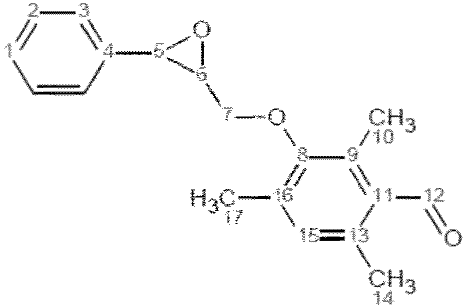 | | |
|---|---|---|
| | δ ¹H (ppm) | δ ¹³C (ppm) |
| 1/2 | 7.18 à 7.34 | 128.2/128.3 |
| 3 | | 125.5 |
| 4 | / | 136.2 |
| 5 | 3.81 | 55.9 |
| 6 | 3.35 | 60.2 |
| 7 | 3.84 et 4.04 | 72.5 |
| 8 | / | 153.8 |
| 9/13/16 | / | 132/133.7/136.7 |
| 10 | 2.5 | 12.2 |
| 11 | / | 131.5 |
| 12 | 10.48 | 193 |
| 14 | 2.48 | 19.8 |
| 15 | 6.87 | 131.8 |
| 17 | 2.28 | 16.6 |

### Solvant CDCl₃

### Synthèse de 2,4,6-triméthyl-3-((3-phényloxiran-2-yl)méthoxy)benzaldéhyde oxime :

A une solution de 2,4,6-triméthyl-3-((3-phényloxiran-2-yl)méthoxy)benzaldéhyde (4,60 g, 0,016 mol) dans de l'alcool éthylique (20 ml) est ajoutée à 45°C une solution d'hydroxylamine (1,43 g, 0,022 mol, 50 % dans l'eau, Aldrich) dans l'alcool éthylique (5 ml). Le milieu réactionnel est agité pendant 1,5 heure à 50°C (T_{bain}). Après retour à température ambiante, de l'eau (3 ml) est ajoutée à la suspension et la suspension est maintenue à -18ºC pendant 2 heures. Le précipité est filtré et lavé sur le filtre par un mélange d'alcool éthylique et d'eau (3 ml / 2 ml et 1 ml / 4 ml) et enfin séché sous pression atmosphérique à température ambiante.

Un solide blanc (3,62 g, rendement massique de 75 %) avec un point de fusion de 125°C est obtenu. La pureté molaire est supérieure à 97 % (RMN ¹H).

**Tableau d'attribution :**

| 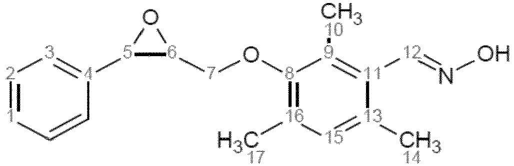 | | |
|---|---|---|
| | δ ¹H (ppm) | δ ¹³C (ppm) |
| 1/2 | 7.21 à 7.35 | 128/128.2 |
| 3 | | 125.4 |
| 4 | / | 136.2 |
| 5 | 3.81 | 56 |
| 6 | 3.35 | 60.3 |
| 7 | 3.85 et 4.02 | 72.2 |
| 8 | / | 153.4 |
| 9 | / | 130.2 |
| 10 | 2.29 | 13.1 |
| 11 | / | 128.2 |
| 12 | 8.31 | 149.6 |
| 13 | / | 132.9 |
| 14 | 2.27 | 20.3 |
| 15 | 6.85 | 130.7 |
| 16 | / | 131 |
| 17 | 2.24 | 16 |

### Solvant CDCl₃

### Synthèse de 2,4,6-triméthyl-3-((3-phényloxiran-2-yl)méthoxy)benzonitrile oxyde (D-4) :

A une solution de 2,4,6-triméthyl-3-((3-phényloxiran-2-yl)méthoxy)benzaldéhyde oxime (10,20 g, 0,033 mol) dans le dichlorométhane (150 ml) refroidi jusqu'à 4°C est ajoutée au goutte à goutte une solution aqueuse de NaOCl dans l'eau (74,4 g Cl/L) (48 ml) pendant 15 minutes. La température du milieu réactionnel reste comprise entre 3 et 5°C. Le milieu réactionnel est ensuite agité pendant 2,5 heures à température 3-5°C. La phase aqueuse est séparée et extraite avec du dichlorométhane (15 ml). Les solutions organiques réunies sont lavées avec de l'eau (3 fois 20 ml). Le solvant est évaporé à pression réduite (T_{bain} 23°C, 22mbar). De l'éther de pétrole (40/60) (60 ml) est ajouté et la suspension est agitée à température ambiante pendant 10-15 minutes. Le précipité est filtré et lavé sur le filtre avec de l'éther de pétrole (2 fois par 20 ml) et enfin séché sous pression atmosphérique à température ambiante. Un solide blanc (8,35 g, rendement massique de 82 %) avec un point de fusion de 64°C est obtenu. La pureté molaire est supérieure à 98 % (RMN ¹H).

**Tableau d'attribution :**

| 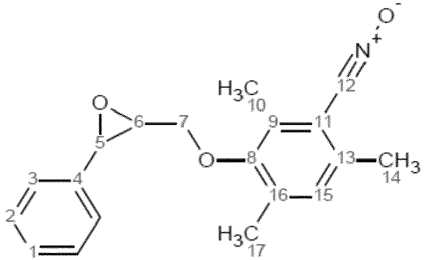 | | |
|---|---|---|
| | δ ¹H (ppm) | δ ¹³C (ppm) |
| 1/2 | 7.21 à 7.35 | 128.2/128.4 |
| 3 | | 125.4 |
| 4 | / | 136.1 |
| 5 | 3.79 | 55.8 |
| 6 | 3.33 | 60.1 |
| 7 | 3.82 et 4.05 | 72.5 |
| 8 | / | 153.3 |
| 9/13/16 | / | 130.3/134.4/137.3 |
| 10 | 2.36 | 14.6 |
| 11 | / | 112.8 |
| 12 | / | / |
| 14 | 2.33 | 20.1 |
| 15 | 6.87 | 130.2 |
| 17 | 2.25 | 16.4 |

### Solvant CDCl₃

### II.3-Greffage des composés 1,3-dipolaires :

Comme agent de greffage, on utilise les composés 1,3-dipolaires D-1 à D-4 dont la synthèse est décrite ci-dessus dans le paragraphe II.2.

Les polymères de départ sont les élastomères suivants :
- E1 : un copolymère de 1,3-butadiène et de styrène (SBR) contenant 26% d'unité styrène et 56% d'unité butadiène-1,2,
- E2 : un copolymère de 1,3-butadiène et de styrène (SBR) contenant 26% d'unité styrène et 24% d'unité butadiène-1,2,
- E3 : un polyisoprène de synthèse fort taux de cis « NIPOL2200 » de Nippon Zeon
- E4 : le caoutchouc naturel.

Les élastomères sont modifiés selon le mode opératoire suivant :
On incorpore le composé 1,3-dipolaire à l'élastomère à l'aide d'un mélangeur interne (outil à cylindre) à 30°C, le taux de composé ajouté est de 0.5 mole pour 100 moles d'unités monomères diéniques de l'élastomère. Le mélange est homogénéisé en 15 passes portefeuille. Cette phase de mélangeage est suivie d'un traitement thermique à 120°C pendant 10 minutes sous presse à 10 bars de pression.

Le rendement de greffage déterminé par analyse RMN pour chacun des élastomères et des composés 1,3-dipolaire figure dans le tableau suivant :

| Rendement | D-1 | D-2 | D-3 |
|---|---|---|---|
| E1 | 75% | 100% | 100% |
| E2 | 100% | 100% | 90% |
| E3 | 38% | 100% | 52% |
| E4 | 30% | 48% | 40% |

### II.4-Préparation des compositions de caoutchouc :

On prépare cinq compositions de caoutchouc, respectivement T, C-1, C-2, C-3 et C-4 dont la formulation (en pce) figure dans le tableau I.

L'élastomère de la composition T est un élastomère non modifié, en l'espèce l'élastomère de départ E1 utilisé pour préparer les élastomères modifiés des compositions 1 à 4.

La composition T est une composition témoin, puisqu'elle contient l'élastomère diénique de départ (non modifié).

L'élastomère de la composition C-n (n allant de 1 à 4) est l'élastomère E1 modifié conformément au paragraphe II-3 par le composé 1,3-dipolaires D-n.

La composition C-1 est une composition comparative, puisque le composé 1,3-dipolaire utilisé pour modifier l'élastomère de la composition n'est pas conforme à l'invention, le deuxième membre du cycle époxyde n'étant ni un carbone tertiaire, ni un carbone quaternaire.

Les compositions C-2 à C-4 sont conformes à l'invention, puisque chacun des composés 1,3-dipolaires utilisés pour modifier l'élastomère des compositions est conforme à l'invention par la présence d'un carbone tertiaire (compositions 3 et 4) ou quaternaire (composition 2) autre que l'atome de carbone assurant le rattachement au dipôle.

Les compositions de caoutchouc sont préparées selon le mode opératoire suivant :
Dans un mélangeur interne, dont la température de cuve initiale est d'environ 80°C, on introduit l'élastomère et on le malaxe pendant environ une minute. Ensuite, on introduit la charge renforçante, le silane, puis, après une à deux minutes de malaxage, les divers autres ingrédients à l'exception du système de vulcanisation. On conduit alors un travail thermomécanique (phase non-productive) en une étape (durée totale du malaxage égale à environ 5 minutes), jusqu'à atteindre une température maximale de "tombée" de 145°C. On récupère le mélange ainsi obtenu, on le refroidit puis on ajoute le système de vulcanisation (soufre) sur un mélangeur externe (homo-finisseur) à 25°C, en mélangeant le tout (phase productive) pendant environ 5 à 6 minutes. Le mélange est ensuite calandré sous forme de plaques (épaisseur de 2 à 3mm) pour la mesure des propriétés de traction et des propriétés dynamiques. Le mélange est ensuite vulcanisé, ses propriétés rhéométriques et ses propriétés à cuit sont mesurées.

Les résultats figurent dans le tableau II. Les résultats sont indiqués en base 100 par rapport à la composition témoin (T) : la valeur indiquée pour une composition étant le rapport entre la valeur mesurée sur la composition et la valeur mesurée sur la composition témoin.

Les compositions C-2 à C-4 vulcanisées présentent un allongement à la rupture et une contrainte à la rupture améliorées par rapport à la composition C-1. Ces résultats sont obtenus sans être au détriment des propriétés d'hystérèse, puisque les valeurs de ΔG^{∗} et de Tan(δ) max restent très inférieures à celle de la composition témoin (T). Les valeurs de ΔC qui sont plus faibles que celle de la composition T corroborent une amélioration de l'interaction entre l'élastomère et la charge renforçante.

On observe aussi que les valeurs de Cmin des compositions C-2 à C-4 sont inférieures à celle de la composition C-1, ce qui indique une diminution de la viscosité à cru (avant vulcanisation) des compositions et augure une mise en œuvre au moins aussi aisée des compositions C-2 à C-4 que la composition T. Ce résultat est d'autant plus surprenant qu'on a constaté par ailleurs une amélioration de l'interaction entre l'élastomère et la charge renforçante.

En résumé, comparativement aux compositions non conformes à l'invention, les compositions de caoutchouc conformes à l'invention sont caractérisées par un compromis amélioré entre les propriétés rupture, les propriétés d'hystérèse et les propriétés de mise en œuvre. L'utilisation des composés 1,3-dipolaires conformes à l'invention dans la confection des compositions de caoutchouc, notamment dans l'étape de modification de l'élastomère de la composition de caoutchouc, permet donc d'améliorer substantiellement leurs propriétés.

**Tableau I**

| Composition | T | C-1 | C-2 | C-3 | C-4 |
|---|---|---|---|---|---|
| E1 | 100 | | | | |
| E1 modifié avec D-1 | | 100 | | | |
| E1 modifié avec D-2 | | | 100 | | |
| E1 modifié avec D-3 | | | | 100 | |
| E1 modifié avec D-4 | | | | | 100 |
| Silice (1) | 60 | 60 | 60 | 60 | 60 |
| Silane (2) | 4,8 | 4,8 | 4,8 | 4,8 | 4,8 |
| Antioxydant (3) | 3 | 3 | 3 | 3 | 3 |
| Paraffine (4) | 1 | 1 | 1 | 1 | 1 |
| ZnO (5) | 2,7 | 2,7 | 2,7 | 2,7 | 2,7 |
| Acide stéarique | 2,5 | 2,5 | 2,5 | 2,5 | 2,5 |
| CBS (6) | 1,8 | 1,8 | 1,8 | 1,8 | 1,8 |
| Soufre | 1,5 | 1,5 | 1,5 | 1,5 | 1,5 |

| | | | | | |
|---|---|---|---|---|---|
| (1) Silice « 160 MP » commercialisée par Solvay (2) TESPT commercialisé par Evonik sous la référence « SI69» (3) N-(l,3-diméthylbutyl)-N'-phényl-p-phénylènediamine de la société Flexsys (4) Agent de mise en œuvre Paraffine 6266 (5) Oxyde de zinc (6) N-cylohexyl-2-benzothiazol-sulfénamide (« Santocure » de la société Flexsys) | | | | | |

**Tableau II**

| Composition | T | C-1 | C-2 | C-3 | C-4 |
|---|---|---|---|---|---|
| Allongement rupture | 100 | 58 | 84 | 68 | 74 |
| Contrainte rupture | 100 | 90 | 111 | 97 | 97 |
| ΔG^{∗} | 100 | 71 | 79 | 88 | 88 |
| Tan(δ) max | 100 | 73 | 73 | 81 | 73 |
| C min | 100 | 110 | 74 | 74 | 84 |
| ΔC | 100 | 68 | 76 | 94 | 66 |

## Revendications

1. Composé 1,3-dipolaire comportant un groupe époxyde, le groupe époxyde étant un cycle éther à 3 membres dans lequel un premier membre est un atome de carbone présentant un rattachement au dipôle du composé 1,3-dipolaire et un deuxième membre est un carbone tertiaire ou quaternaire, lequel composé est un monooxyde de nitrile aromatique.

2. Composé 1,3-dipolaire selon la revendication 1, dans lequel le groupe époxyde est de formule (I) 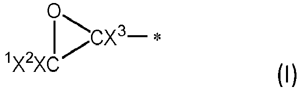 dans laquelle
- ^{∗} représente un rattachement au dipôle,
- X¹, X² et X³, identiques ou différents, représentent un atome d'hydrogène ou un groupe substituant, et
- au moins un des X¹, X² et X³ est différent d'un atome d'hydrogène.

3. Composé 1,3-dipolaire selon la revendication 2 dans lequel le groupe substituant est un alkyle ou un aryle.

4. Composé 1,3-dipolaire selon l'une quelconque des revendications 1 à 3 qui comprend un noyau benzénique, lequel noyau benzénique est substitué par le dipôle du composé 1,3-dipolaire.

5. Composé 1,3-dipolaire selon la revendication 4 dans lequel le noyau benzénique est substitué en ortho du dipôle.

6. Composé 1,3-dipolaire selon l'une quelconque des revendications 1 à 5 lequel composé contient un motif de formule (II) 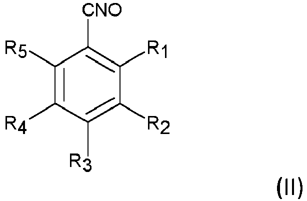 dans laquelle :
- quatre des cinq symboles R1 à R5, identiques ou différents, sont chacun un atome ou un groupe d'atomes, et le cinquième symbole représente une chaîne carbonée permettant le rattachement au groupe époxyde, sachant qu'au moins un des R1 et R5 est différent d'un atome d'hydrogène.

7. Composé 1,3-dipolaire selon la revendication 6 dans lequel R1, R3 et R5 représentent chacun un groupe hydrocarboné, de préférence alkyle, de manière plus préférentielle méthyle ou éthyle.

8. Composé 1,3-dipolaire selon l'une quelconque des revendications 6 à 7 dans lequel le cinquième symbole représente une chaîne carbonée interrompue par un ou plusieurs hétéroatomes, de préférence oxygène.

9. Composé 1,3-dipolaire selon l'une quelconque des revendications 6 à 8 dans lequel le cinquième symbole comprend un motif -CH₂O-, le groupe méthylène étant attaché au groupe époxyde.

10. Composé 1,3-dipolaire selon l'une quelconque des revendications 1 à 9 ayant pour formule l'une des formules (III), (IV) ou (V)

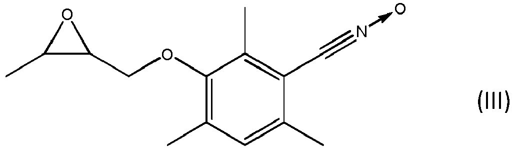

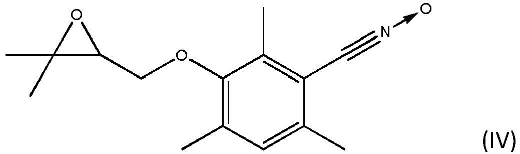

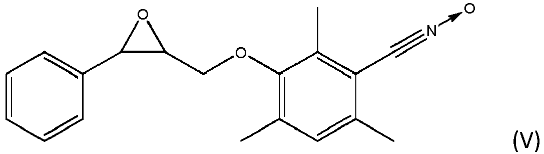

11. Procédé pour modifier un polymère diénique par une réaction de greffage qui comprend la réaction d'un polymère diénique de départ et d'un composé 1,3-dipolaire défini à l'une quelconque des revendications 1 à 10.

12. Polymère susceptible d'être obtenu par le procédé défini à la revendication 11.

13. Polymère selon la revendication 12, lequel polymère est un élastomère diénique.

14. Composition de caoutchouc qui comprend une charge renforçante, un système de réticulation et un élastomère diénique défini à la revendication 13.

15. Pneumatique qui comprend une composition de caoutchouc selon la revendication 14.

## Patentansprüche

1. 1,3-Dipolare Verbindung mit einer Epoxidgruppe, wobei es sich bei der Epoxidgruppe um einen 3-gliedrigen Etherring handelt, in dem ein erstes Glied ein Kohlenstoffatom mit einer Anbindung an den Dipol der 1,3-dipolaren Verbindung ist und ein zweites Glied ein tertiäres oder quaternäres Kohlenstoffatom ist, wobei es sich bei der Verbindung um ein aromatisches Nitrilmonooxid handelt.

2. 1,3-Dipolare Verbindung nach Anspruch 1, wobei die Epoxidgruppe die Formel (I) aufweist: 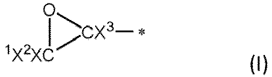 wobei
- * für eine Anbindung an den Dipol steht,
- X¹, X² und X³ gleich oder verschieden sind und für ein Wasserstoffatom oder eine Substituentengruppe stehen und
- mindestens eines von X¹, X² und X³ von einem Wasserstoffatom verschieden ist.

3. 1,3-Dipolare Verbindung nach Anspruch 2, wobei es sich bei der Substituentengruppe um ein Alkyl oder ein Aryl handelt.

4. 1,3-Dipolare Verbindung nach einem der Ansprüche 1 bis 3, die einen durch den Dipol der 1,3-dipolaren Verbindung substituierten Benzolkern umfasst.

5. 1,3-Dipolare Verbindung nach Anspruch 4, wobei der Benzolkern in ortho-Position zum Dipol substituiert ist.

6. 1,3-Dipolare Verbindung nach einem der Ansprüche 1 bis 5, wobei die Verbindung eine Einheit der Formel (II) umfasst: 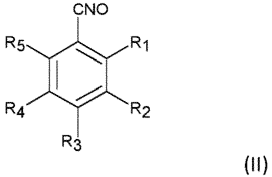 wobei:
- vier der fünf Symbole R₁ bis R₅ gleich oder verschieden sind und jeweils für ein Atom oder eine Gruppe von Atomen stehen und das fünfte Symbol für eine die Anbindung an die Epoxidgruppe ermöglichende Kohlenstoffkette steht, mit der Maßgabe, dass mindestens eines von R₁ bis R₅ von einem Wasserstoffatom verschieden ist.

7. 1,3-Dipolare Verbindung nach Anspruch 6, wobei R₁, R₃ und R₅ jeweils für eine Kohlenwasserstoffgruppe, vorzugsweise eine Alkylgruppe, weiter bevorzugt eine Methyl- oder Ethylgruppe, stehen.

8. 1,3-Dipolare Verbindung nach einem der Ansprüche 6 bis 7, wobei das fünfte Symbol für eine durch ein oder mehrere Heteroatome, vorzugsweise Sauerstoff, unterbrochene Kohlenstoffkette steht.

9. 1,3-Dipolare Verbindung nach einem der Ansprüche 6 bis 8, wobei das fünfte Symbol eine -CH₂O-Einheit umfasst, wobei die Methylengruppe an die Epoxidgruppe gebunden ist.

10. 1,3-Dipolare Verbindung nach einem der Ansprüche 1 bis 9, die als Formel eine der Formeln (III), (IV) oder (V) aufweist:

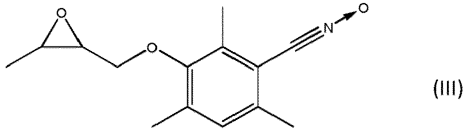

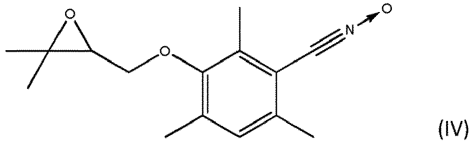

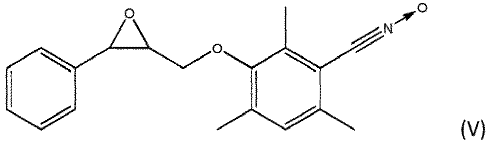

11. Verfahren zur Modifizierung eines Dienpolymers durch eine Pfropfreaktion, die die Umsetzung eines Ausgangsdienpolymers und einer 1,3-dipolaren Verbindung gemäß einem der Ansprüche 1 bis 10 umfasst.

12. Polymer, das durch das Verfahren gemäß Anspruch 11 erhältlich ist.

13. Polymer nach Anspruch 12, wobei es sich bei dem Polymer um ein Dienelastomer handelt.

14. Kautschukzusammensetzung, die einen verstärkenden Füllstoff, ein Vernetzungssystem und ein Dienelastomer gemäß Anspruch 13 umfasst.

15. Reifen, der eine Kautschukzusammensetzung gemäß Anspruch 14 umfasst.

## Claims

1. 1,3-Dipolar compound including an epoxide group, the epoxide group being a 3-membered ether ring in which a first member is a carbon atom having an attachment to the dipole of the 1,3-dipolar compound and a second member is a tertiary or quaternary carbon, which compound is an aromatic nitrile monoxide.

2. 1,3-Dipolar compound according to Claim 1, in which the epoxide group is of formula (I) 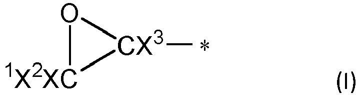 in which:
- ^{∗} represents an attachment to the dipole,
- X¹, X² and X³, which may be identical or different, represent a hydrogen atom or a substituent group, and
- at least one from among X¹, X² and X³ is other than a hydrogen atom.

3. 1,3-Dipolar compound according to Claim 2, in which the substituent group is an alkyl or an aryl.

4. 1,3-Dipolar compound according to any one of Claims 1 to 3, which comprises a benzene nucleus, which benzene nucleus is substituted with the dipole of the 1,3-dipolar compound.

5. 1,3-Dipolar compound according to Claim 4, in which the benzene nucleus is substituted ortho to the dipole.

6. 1,3-Dipolar compound according to any one of Claims 1 to 5, which compound contains a unit of formula (II) 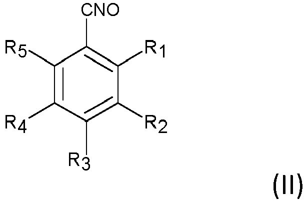 in which:
- four of the five symbols R1 to R5, which may be identical or different, are each an atom or a group of atoms, and the fifth symbol represents a carbon-based chain for attachment to the epoxide group, given that at least one from among R1 and R5 is other than a hydrogen atom.

7. 1,3-Dipolar compound according to Claim 6, in which R1, R3 and R5 each represent a hydrocarbon-based group, preferably alkyl, more preferentially methyl or ethyl.

8. 1,3-Dipolar compound according to either one of Claims 6 and 7, in which the fifth symbol represents a carbon-based chain interrupted with one or more heteroatoms, preferably oxygen.

9. 1,3-Dipolar compound according to any one of Claims 6 to 8, in which the fifth symbol comprises a -CH₂O- group, the methylene group being attached to the epoxide group.

10. 1,3-Dipolar compound according to any one of Claims 1 to 9, having as formula one of the formulae (III), (IV) or (V)

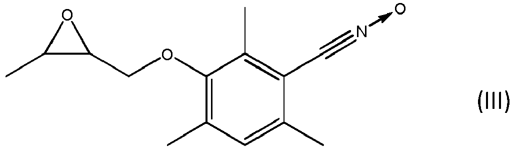

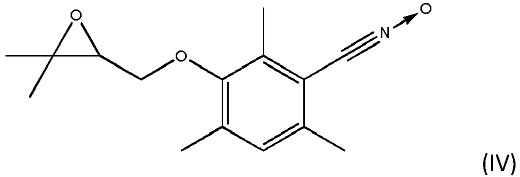

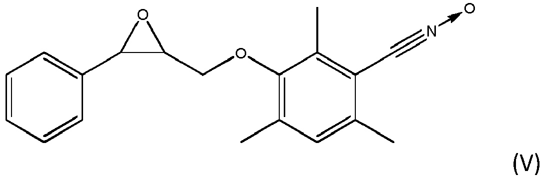

11. Process for modifying a diene polymer via a grafting reaction, which comprises the reaction of a starting diene polymer and of a 1,3-dipolar compound defined in any one of Claims 1 to 10.

12. Polymer which may be obtained via the process defined in Claim 11.

13. Polymer according to Claim 12, which polymer is a diene elastomer.

14. Rubber composition which comprises a reinforcing filler, a crosslinking system and a diene elastomer defined in Claim 13.

15. Tyre which comprises a rubber composition according to Claim 14.
